# FASCICULE DE BREVET EUROPEEN

(11) **EP 3 022 283 B1**
(45) Date de publication et mention de la délivrance du brevet: **09.10.2019**
(21) Numéro de dépôt: 14739162.7
(22) Date de dépôt: 15.07.2014
(51) Int. Cl.: C12M 1/00, C12M 1/12, C12M 1/26

(54) **PROCÉDÉ ET INSTALLATION DE PRODUCTION DE MICRO-ALGUES**
VERFAHREN UND VORRICHTUNG ZUR PRODUKTION VON MIKROALGEN
METHOD AND UNIT FOR PRODUCING MICROALGAE

(30) Priorité: 15.07.2013 FR 1356955
(43) Date de publication de la demande: 25.05.2016
(73) Titulaire: INRIA - Institut National de Recherche en Informatique et en Automatique, 78150 Le Chesnay (FR); Centre National de la Recherche Scientifique (C.N.R.S.), 75016 Paris (FR); Centrale Supélec, 92295 Chatenay Malabry (FR); Université Pierre et Marie Curie (Paris 6), 75005 Paris (FR)
(72) Inventeur: BERNARD, Olivier, F-06510 Carros (FR); LOPES, Filipa, F-75015 Paris (FR); PRUVOST, Eric, F-06100 Nice (FR); SCIANDRA, Antoine, F-06230 Villefranche Sur Mer (FR)
(74) Mandataire: Palacci, Jeremie
(86) Numéro de dépôt international: PCT/EP2014/065126
(87) Numéro de publication internationale: WO 2015/007724

(56) Documents cités:
- WO-A1-2012/171123
- WO-A1-2013/071364
- US-A1- 2010 144 017

## Description

La présente invention concerne un procédé de production de micro-algues sur un support monté mobile essentiellement dans un milieu aqueux contenu dans un bassin comportant une succession de phases d'exposition à la lumière solaire des micro-algues se développant sur le support et de phases de séjour dans l'ombre, l'intensité lumineuse reçue dans l'ombre étant de moins de 50% de l'intensité lumineuse moyenne reçue lors des phases d'exposition à la lumière solaire.

La production en milieu aqueux de micro-algues ou de tout autre microorganisme photosynthétique est utilisée pour pourvoir aux besoins des marchés de la cosmétique, l'industrie pharmaceutique, l'aquaculture, la production d'alicaments ou de compléments alimentaires, ainsi que la production de bioénergie, en exploitant la capacité des micro-algues à capter l'énergie lumineuse pour fixer du carbone inorganique (principalement sous forme de dioxyde de carbone ou bicarbonate).

Les micro-algues sont le plus couramment cultivées en suspension dans un milieu liquide dont la surface est exposée au soleil. Pour s'assurer que chaque cellule puisse être, au moins par intermittence, exposée à la lumière, le milieu est brassé, par exemple à l'aide de roues à aubes. Il existe également des photo-bioréacteurs fermés, constitués d'une enceinte transparente munie d'un dispositif d'agitation ou de moyens de mise en circulation du flux liquide entre deux parois permettant l'exposition à une source lumineuse.

Quelles qu'elles soient, ces installations, qui nécessitent le déplacement du milieu de culture, sont très consommatrices d'énergie. Par ailleurs, la récolte des micro-algues requiert elle-même une énergie importante, en raison de leur faible concentration et de la faible différence de leur densité avec celle du milieu, qui rend leur séparation délicate. Cette séparation est couramment opérée par centrifugation, également consommatrice d'énergie.

Les micro-algues peuvent également se développer sur un support mobile tel qu'un support refermé en boucle, circulant sur des rouleaux partiellement ou totalement immergés dans un milieu aqueux.

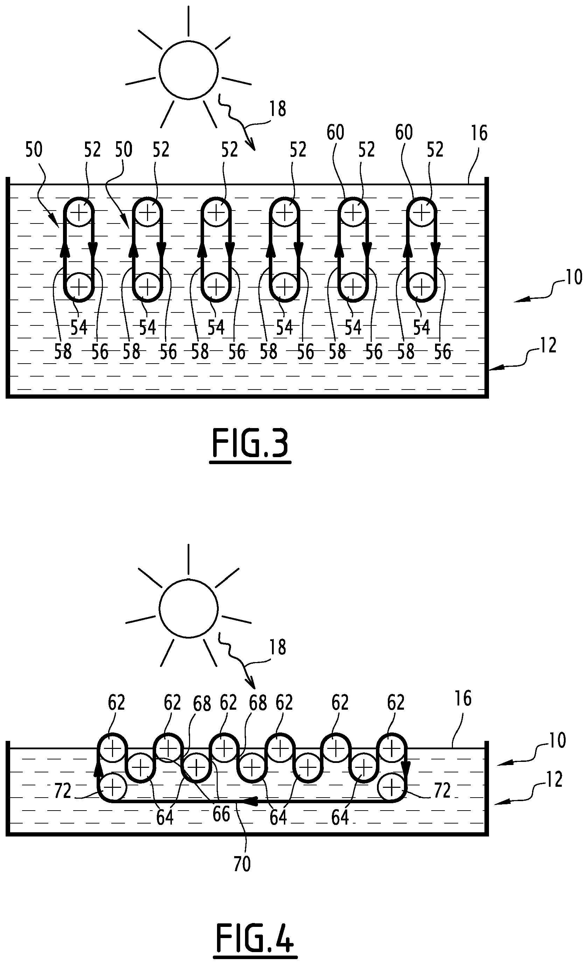

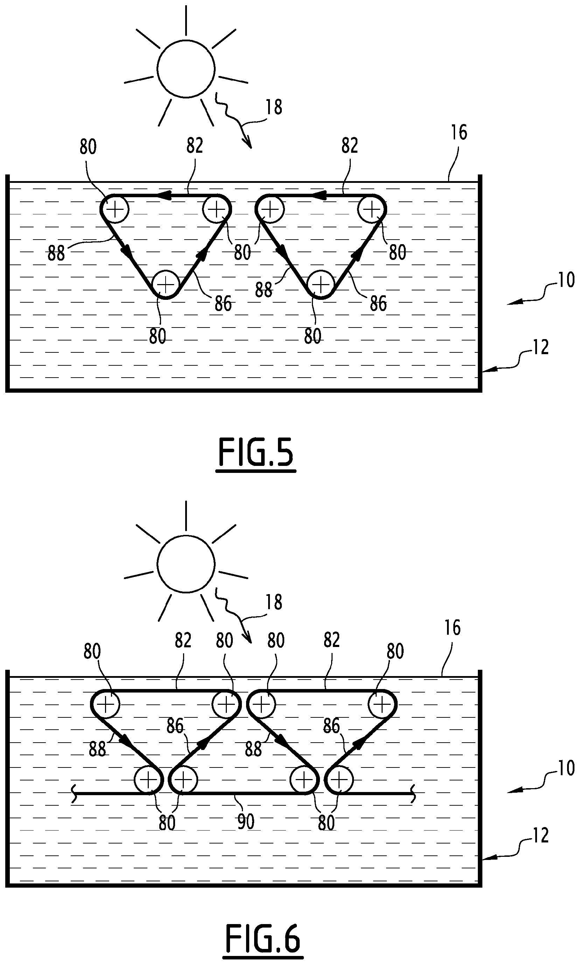

Une telle installation, qui cherche à maximiser l'exposition à un éclairage artificiel ou naturel du biofilm de microalgues à la surface du support afin d'en optimiser la croissance, est décrite par exemple dans le document AU 2012 101 593. Les micro-algues sont exposées à la lumière essentiellement lorsque la portion du support sur lequel elles s'accumulent se trouve émergé. La combinaison de sources lumineuses naturelle et artificielle (diodes électroluminescentes) permet de maximiser l'intensité lumineuse, notamment lors des variations d'intensité lumineuse du soleil, comme indiqué au paragraphe WO2013071364 décrit un photobioréacteur comprenant une bande mobile sur laquelle sont cultivées des algues (abstrait, [0063]). Des rouleaux transportent la bande à travers du milieu de culture (fig. 3, [0080]) et à travers une zone d'illumination (fig. 5, [0088]). Les algues peuvent être cultivés dans l'obscurité ou dans la lumière, ou dans des phases alternantes de lumière et d'ombre, cela dépend de l'algue cultivée ([0059], [0123]).

Même si ces procédés permettent une récolte facilitée et moins énergivore par rapport aux procédés de production de micro-algues en suspension, leurs rendements restent modestes, notamment aux fortes lumières.

L'invention a pour but de proposer un procédé de production de micro-algues qui améliore le rendement notamment aux fortes intensités lumineuses.

A cet effet, l'invention a pour objet un procédé de production de micro-algues caractérisé en ce que la durée totale des phases de séjour dans l'ombre est supérieure de 50% à la durée totale des phases d'exposition à la lumière solaire.

Suivant des modes particuliers de réalisation, l'invention comporte une ou plusieurs des caractéristiques suivantes :
- la durée totale des phases de séjour dans l'ombre est comprise entre 2 et 10 fois la durée totale des phases d'exposition à la lumière ;
- la durée de chaque phase d'exposition à la lumière est comprise entre 5 secondes et 10 minutes ;
- le support circule en boucle entre des rouleaux, et la vitesse du support est comprise entre 1 cm/s et 1 m/s ;
- la dose totale d'éclairement des micro-algues pendant chaque phase d'exposition est comprise entre 1 et 60 millimoles photons/m² ;
- les micro-algues font partie de l'une ou plusieurs des espèces comprises dans le groupe consistant en *Botryoccocus sp., Porhyridium sp., Cylindrotheca sp., Navicula sp. Haslea sp. et Chlorella sp.* ; et
- les micro-algues sont maintenues au moins 90% du temps dans le milieu aqueux.

L'invention concerne également une installation de production de micro-algues comportant une ou plusieurs des caractéristiques suivantes :
- ladite installation de production de micro-algues comporte :
   - un bassin ;
   - un support monté mobile dans un milieu aqueux contenu dans le bassin, et
   - des moyens d'entrainement et de guidage du support successivement sur des tronçons d'exposition des micro-algues sur le support à la lumière solaire et sur des tronçons de repos dans l'ombre, l'agencement étant tel que l'intensité lumineuse reçue lors des déplacements sur les tronçons de repos dans l'ombre est de moins de 50% de l'intensité lumineuse moyenne reçue lors des déplacements sur les tronçons d'exposition à la lumière solaire, caractérisée en ce que les moyens d'entrainement et de guidage du support sont adaptés pour que la durée totale des déplacements sur les tronçons de repos dans l'ombre est supérieure de 50% à la durée totale des déplacements sur les tronçons d'exposition à la lumière solaire ;
- le support est replié en boucle et le support est guidé suivant des tronçons superposés, le tronçon supérieur formant un tronçon d'exposition disposé au-dessus des autres tronçons formant des tronçons de repos situés dans l'ombre du tronçon d'exposition ;
- le support est replié en boucle et comporte des moyens de guidage du support suivant des tronçons verticaux ascendants et descendants sensiblement perpendiculairement à la surface du milieu aqueux contenu dans les bassins disposés dans l'ombre ; et
- le support est une bande de Möbius.

L'invention sera mieux comprise à la lecture de la description qui va suivre, donnée uniquement à titre d'exemple, et faite en se référant aux dessins sur lesquels :
- la figure 1 est un schéma d'une installation vue de côté de production de micro-algues selon l'invention ;
- la figure 2 est une vue identique à celle de la figure 1 d'une variante de réalisation ;
- la figure 3 est une vue schématique simplifiée de côté d'un autre mode de réalisation d'une installation de production de micro-algues selon l'invention ;
- la figure 4 est une vue identique à celle de la figure 3 d'une variante de réalisation ;
- la figure 5 est une vue schématique simplifiée de côté d'encore un autre mode de réalisation d'une installation de production de micro-algues selon l'invention ;
- la figure 6 est une vue identique à celle de la figure 5 d'une variante de réalisation ;
- la figure 7 est une vue schématique simplifiée de côté d'un dernier mode de réalisation d'une installation de production de micro-algues selon l'invention ;
- la figure 8 est une vue en coupe longitudinale d'un exemple de réalisation d'un rouleau d'une installation des figures précédentes ; et
- la figure 9 est une vue identique à celle de la figure 8 d'une variante de réalisation.

L'invention décrite dans la suite est, quel que soit le mode de réalisation, particulièrement adaptée à des zones soumises à de fortes intensités lumineuses produites par le rayonnement solaire. Au sens de la présente demande, de fortes intensités lumineuses sont supérieures à 300 micromoles photons/m²/s.

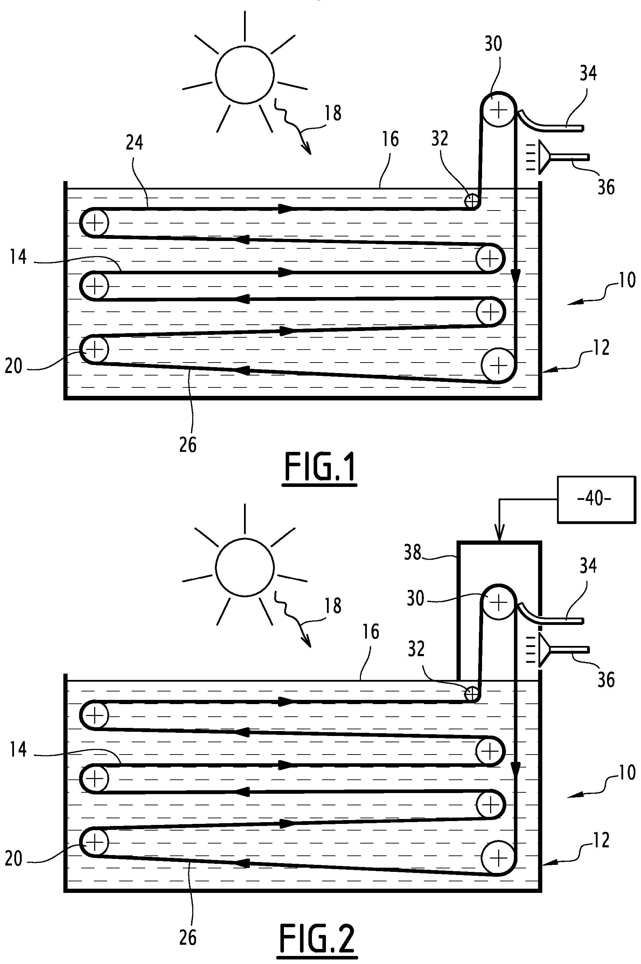

L'installation 10 représentée sur la figure 1 comporte essentiellement un bassin 12 et un support 14 circulant dans le bassin 12 et sur lequel se développent des micro-algues formant un biofilm.

Les micro-algues sont des cellules de tailles comprises entre 1 à 100 micromètres. Ces micro-algues font partie de l'une ou plusieurs des espèces parmi *Botryoccocus braunii, Porhyridium cruentum,* et des espèces épipéliques telles que *Cylindrotheca closterium* ou *Navicula salinarum.* Les espèces ainsi cultivées, seules ou en consortium avec d'autres algues, telles que *Chlorella sorokiniana* et/ou des bactéries sont avantageusement utilisées pour dégrader ou fixer des éléments dans l'eau.

Le bassin 12 est ouvert dans sa partie supérieure. Sa surface, notée 16, est directement exposée au rayonnement solaire 18 et s'étend sur toute la surface ouverte du bassin 12. Elle a une aire comprise entre 0,1 m² et 10 000 m² et de préférence comprise entre 10 et 1 000 m² L'installation est dépourvue de toute source lumineuse autre que le soleil pour l'exposition des micro-algues.

Le bassin 12 est constitué, par exemple, d'une cuve maçonnée, ou encore d'une étendue d'eau naturelle, telle qu'un lac ou un étang, voire une baie marine. En variante, le bassin est un bassin de traitement d'eau d'une station d'épuration, notamment adapté au traitement de l'azote et du phosphore inorganiques.

De préférence, l'eau contenue dans le bassin est renouvelée en permanence.

Lorsqu'il s'agit d'une cuve maçonnée, le bassin a une profondeur comprise entre 10 cm et 150 cm et de préférence entre 50 cm et 1 m. De préférence, la profondeur est supérieure à 50 cm.

Le support 14 est formé d'une bande renfermée en boucle sur elle-même, et guidée par un ensemble de rouleaux de retournement et de guidage 20. L'un des rouleaux, au moins, est motorisé pour assurer l'entraînement du support 14.

Le support est formé par exemple d'un tapis, assurant la résistance mécanique, recouvert sur une face d'une nappe tissée de polymère ou de tout autre support souple. Le support du biofilm est préférentiellement un support hydrophobe et rugueux présentant des cavités ou microcavités. Il présente une flexibilité suffisante pour supporter le passage sur les rouleaux. Il résiste à la lumière et, en particulier, aux rayons ultra-violets. Le matériau est choisi pour que son éventuelle détérioration n'affecte pas l'activité biologique.

Des matériaux tels que le coton, la toile de jute, le polyéthylène, le polyuréthane conviennent en tant que nappe de support du biofilm. Des nappes en polyester et polyuréthane utilisés en agroalimentaire colonisés par des bactéries sont notamment adaptées.

Avantageusement, des bio-polymères sont utilisés pour former la nappe de support du biofilm.

Le support présente avantageusement une largeur comprise entre 0,1 m et 2 m. Sa longueur totale est par exemple comprise entre 1 m et 5 000 m.

Le rapport entre le volume du milieu liquide contenu dans le bassin 12, la surface ouverte 16 et la surface du support est déterminé afin d'éviter un échauffement trop important du bassin sous l'effet du rayonnement solaire, afin que la température du milieu reste comprise entre 20°C et 35°C.

Dans le mode de réalisation de la figure 1, deux rangées verticales de rouleaux sont disposées aux deux extrémités opposées du bassin 12.

Les rouleaux sont disposés avec leurs axes parallèles à la surface de l'eau 16. Les axes des rouleaux sont parallèles entre eux. Les rouleaux sont agencés les uns au-dessus des autres de part et d'autre du bassin de sorte que le support circule sur des tronçons généralement horizontaux d'un rouleau vers un autre rouleau du bord opposé, le support subissant un retournement sur chaque rouleau.

L'agencement est tel que le support circule suivant des tronçons superposés et parallèles les uns aux autres et à la surface de l'eau 16. Ainsi, le support circule suivant un parcours en boustrophédon. Le tronçon de circulation du support disposé immédiatement au-dessous de la surface 16 forme un tronçon 24 d'exposition des micro-algues au rayonnement solaire à travers la surface 16.

Le tronçon d'exposition 24 est situé à une profondeur comprise entre 3 cm et 20 cm par rapport à la surface 16. La profondeur est choisie avantageusement de telle sorte que le support soit le plus proche possible de la surface 16, tout en limitant les interactions avec les vaguelettes de surface. L'intensité lumineuse sur le tronçon d'exposition 24 est alors avantageusement comprise entre 100 et 400 micromoles photons/m²/s.

Sur son tronçon d'exposition 24, le support recouvre les autres tronçons de circulation du support disposés à un niveau inférieur. Ces tronçons de circulation inférieurs, formant des tronçons de repos 26 sont masqués du rayonnement solaire direct par la présence du tronçon d'exposition 24 généralement opaque, et se trouvent ainsi dans l'ombre.

Au sens du brevet, l'ombre constitue une partie du milieu dans lequel l'intensité lumineuse résultant de la lumière solaire est réduite à une valeur inférieure à 50% de l'intensité lumineuse moyenne reçue sur le tronçon d'exposition 24.

Ainsi, l'intensité lumineuse dans l'ombre à laquelle sont soumises les micro-algues sur les tronçons de repos 26 est inférieure à 100 micromoles photons/m²/s et de préférence inférieure à 10 micromoles photons/m²/s. Dans le mode de réalisation représenté, les tronçons de repos 26, suivant lesquels le support est dans l'ombre, sont au nombre de cinq. Le support circule avantageusement du fond du bassin 12 vers la surface supérieure 16 en suivant les différents tronçons de repos 26, avant de parcourir le tronçon d'exposition 24.

La vitesse de circulation du support est de préférence comprise entre 1 cm/s et 1 m/s. La vitesse est avantageusement adaptée à la lumière solaire directe reçue par le tronçon 24, de sorte que la dose d'éclairement reçue par le biofilm lors de chaque passage sur un tronçon d'exposition 24 ne dépasse pas 60 millimoles photons/m². Ainsi, le temps de passage sur le tronçon d'exposition 24 est inférieur à 10 minutes et généralement compris entre 1 et 3 minutes.

La longueur du tronçon d'exposition 24 est comprise entre 0,1 m et 1 000 m. Sa longueur et sa vitesse sont telles que la durée des phases d'exposition des micro-algues à la lumière solaire directe, c'est-à-dire la durée de circulation des micro-algues sur le tronçon d'exposition 24, est comprise, en fonction de l'intensité lumineuse de surface, entre 5 s et 10 mn.

On comprend que, dans l'exemple illustré, les micro-algues séjournent dans une zone d'ombre lors de la circulation sur les tronçons de repos 26 pendant une durée totale environ cinq fois supérieure à la durée totale d'exposition à la lumière lors de la circulation sur le tronçon 24.

De préférence et plus généralement, selon l'invention, la durée totale des phases de séjour dans une zone d'ombre est supérieure de cinquante pourcent (50%) à la durée totale des phases d'exposition à la lumière.

Avantageusement, notamment pour les fortes intensités lumineuses solaires, au-delà de 1 000 micromoles/m²/s, la durée totale des phases de séjour dans une zone d'ombre est comprise entre 2 et 10 fois la durée totale des phases d'exposition à la lumière. Elle est de préférence au moins 5 fois supérieure à la durée totale des phases d'exposition à la lumière.

En outre, de préférence, la durée totale des phases de séjour dans une zone d'ombre est au plus 10 fois supérieure à la durée totale des phases d'exposition à la lumière.

Enfin, de préférence, la valeur de l'intensité lumineuse moyenne reçue par une micro-algue passant par des phases d'exposition et d'ombre successives est inférieure à un seuil, par exemple de 100 micromoles photons/m²/s, lors des périodes d'ensoleillement de l'installation, c'est-à-dire pendant la journée. Cette valeur de moyenne correspond à la valeur moyenne reçue par une micro-algue sur un cycle, lors des périodes d'ensoleillement de l'installation. Il est entendu par « cycle » le passage d'un biofilm tel qu'une micro-algue sur l'ensemble des tronçons de repos 26 et sur le tronçon d'exposition 24 de sorte que le biofilm ne passe qu'une seule fois sur chacun des tronçons 24, 26 considérés.

L'installation comporte en outre un rouleau 30 disposé au-dessus de la surface 16 du bassin. Le support circule sur ce rouleau 30 après s'être engagé sur un rouleau 32 formant un renvoi d'angle disposé à l'extrémité finale du tronçon d'exposition 24. Un racleur déplaçable 34 est monté en regard du rouleau 30 pour assurer, lors des phases de récolte, un raclage du support et un prélèvement des micro-algues de surface.

Le racleur est mis en place à des intervalles de temps permettant de conserver l'épaisseur du biofilm autour d'une valeur moyenne prédéfinie telle que le taux de respiration dans la couche du biofilm en contact avec le support soit sensiblement égal au taux de fixation de carbone inorganique par photosynthèse de cette même couche de contact, de sorte que la quantité de carbone fixé dans la couche de contact du biofilm soit comprise entre la moitié et le double de la quantité de carbone perdu par la respiration des microorganismes du biofilm. Idéalement, la récolte se fait une fois par jour à une fois par semaine de manière à maintenir l'épaisseur du biofilm autour de cette valeur.

En outre, l'installation comporte un pulvérisateur 36 d'éléments nutritifs formant des sources d'azote, de phosphore, de vitamines et de micronutriments sur le tronçon de retour du support 14 dans le bassin 12.

Le temps passé hors du milieu de culture permet de dégazer l'oxygène accumulé dans le biofilm ou à proximité, et d'enrichir le biofilm en dioxyde de carbone.

On constate que l'installation décrite ici, ainsi que le procédé de production de micro-algues qu'elle met en oeuvre, permettent d'éviter un échauffement trop important du biofilm et d'obtenir une forte efficacité photosynthétique, car les micro-algues, n'étant exposée directement au rayonnement solaire que durant un laps de temps modéré, ne sont pas sujettes à la photo-inhibition.

En variante, l'installation comporte des moyens de réglage du ratio entre le temps total d'exposition et le temps total de séjour à l'ombre du biofilm, notamment pour tenir compte de l'intensité lumineuse fournie par le soleil. Ces moyens comportent par exemple des moyens de déplacement parallèlement à la surface 16 d'un rouleau d'extrémité 20 du tronçon d'exposition 24. Le déplacement du rouleau assure un allongement ou un rétrécissement du tronçon d'exposition, augmentant ou réduisant en conséquence le ratio précité.

Pour assurer une tension du support, un autre rouleau d'extrémité d'un tronçon de repos 26 est déplaçable conjointement pour adapter de manière correspondante la longueur du tronçon de retour.

En variante encore, l'installation comporte des moyens de régulation de la vitesse de circulation du support pour augmenter ou diminuer le temps d'exposition.

Suivant un mode particulier de réalisation, le bassin comporte des moyens de réglage de la hauteur d'eau dans le bassin afin de maintenir la température dans une plage de valeurs prédéterminées.

Dans la variante de réalisation, illustrée sur la figure 2, l'installation comporte, outre les éléments prévus sur la figure 1, une cloche 38 d'injection de dioxyde de carbone. La cloche 38 recouvre de manière hermétique le tronçon du support émergeant au-dessus de la surface 16 du bassin. Cette cloche s'ouvre dans le bassin, au-dessous de la surface 16, délimitant ainsi un espace confiné empli d'une atmosphère plus riche en dioxyde de carbone que l'air. L'intérieur de la cloche 38 est relié à une source 40 d'injection de dioxyde de carbone de manière à y assurer une atmosphère riche en dioxyde de carbone. De préférence, la teneur en dioxyde de carbone dans la cloche est supérieure à 3% en rapport volumique.

On comprend que, lors de la circulation du support à l'intérieur de la cloche 38, les micro-algues portées par le support se trouvent mises en contact avec le dioxyde de carbone, permettant ainsi sa capture sous l'action du rayonnement solaire auquel elles ont été préalablement exposées.

Dans les modes de réalisation qui suivent, un tronçon de récolte non représenté tel que celui décrit en regard de la figure 1 est mis en oeuvre. Avantageusement, le rouleau 30 est monté mobile et est maintenu sous le niveau de la surface 16 dans le milieu aqueux hors des phases de récolte et n'est émergé que lors des phases de récolte.

Dans le mode de réalisation de la figure 3, le bassin 12 renferme plusieurs supports 50 refermés en boucle et agencés parallèlement les uns aux autres. Ces supports 50 indépendants sont repliés seulement autour de deux rouleaux de retournement disposés l'un au-dessus de l'autre. L'un des rouleaux est motorisé pour l'entrainement du support. Un rouleau de retournement supérieur 52 est disposé immédiatement au-dessous de la surface 16 alors qu'un rouleau de retournement inférieur 54 est disposé plus en profondeur au droit du rouleau 52.

La bande formant le support circule ainsi essentiellement verticalement entre les deux rouleaux suivant un brin descendant 56 et un brin montant 58. Dans ce mode de réalisation, les micro-algues portées par la surface exposée du support sont soumises à des phases d'exposition à la lumière solaire sur un tronçon d'exposition noté 60 seulement sur le demi-périmètre supérieur du rouleau supérieur 52 lors du retournement du support. Lors des tronçons descendants 56 et ascendants 58 et lors du retournement autour du rouleau inférieur 54, le support est maintenu dans l'ombre, l'exposition éventuelle à la lumière solaire conduisant à une intensité lumineuse faible compte tenu de l'incidence du rayonnement solaire par rapport au support, et de la profondeur plus importante à laquelle se trouve le support.

Dans la variante de réalisation illustrée sur la figure 4, un unique support est disposé entre deux séries de rouleaux supérieurs 62 et inférieurs 64 pour former des tronçons ascendants 66 et descendants 68 successifs. Ainsi, comme dans le mode de réalisation de la figure 1, le support circule suivant un trajet en boustrophédon mais avec des tronçons de circulation rectilignes verticaux, complété par un trajet de retour 70 assuré entre deux rouleaux extrêmes inférieurs 72. Ainsi, le tronçon de retour 72 s'effectue au-dessous des rouleaux inférieurs 64.

Dans cette variante de réalisation, l'axe des rouleaux 62 s'étend sensiblement dans le plan de la surface 16 du bassin de sorte que la moitié supérieure des rouleaux 62 est située hors du milieu aqueux, et est directement soumise au rayonnement solaire, alors que la moitié inférieure des rouleaux 62 est maintenue dans le milieu liquide. Comme précédemment, la bande n'est soumise à l'exposition à la lumière solaire que sur la moitié supérieure des rouleaux 62 constituant le tronçon d'exposition noté 74, la bande circulant pour le reste de son trajet dans l'ombre.

Dans le mode de réalisation de la figure 5, chaque bande est guidée par trois rouleaux 80 délimitant des tronçons de circulation s'étendant suivant les trois côtés d'un triangle équilatéral. L'un des côtés du triangle définissant le tronçon d'exposition, noté 82, s'étend parallèlement à la surface 16 du bassin, les deux autres côtés formant des tronçons de repos ascendants 86 et descendants 88 s'étendant sous le tronçon d'exposition 82, dans l'ombre de celui-ci.

Dans ce mode de réalisation, le temps total de circulation dans une zone d'ombre est le double de la durée totale d'exposition.

Dans la variante de réalisation de la figure 6, les rouleaux inférieurs sont remplacés par des rouleaux permettant la circulation d'une même bande successivement sur les différents tronçons exposés 82 après circulation sur les deux tronçons descendant 88 et ascendant 86, ces deux tronçons étant reliés par un tronçon de liaison inférieur 90, lui aussi disposé dans l'ombre. Un tronçon de retour disposé en profondeur et non représenté assure le retour de la bande d'une extrémité à l'autre du bassin.

Dans ce mode de réalisation, le temps total de circulation dans une zone d'ombre est supérieur au triple de la durée totale d'exposition.

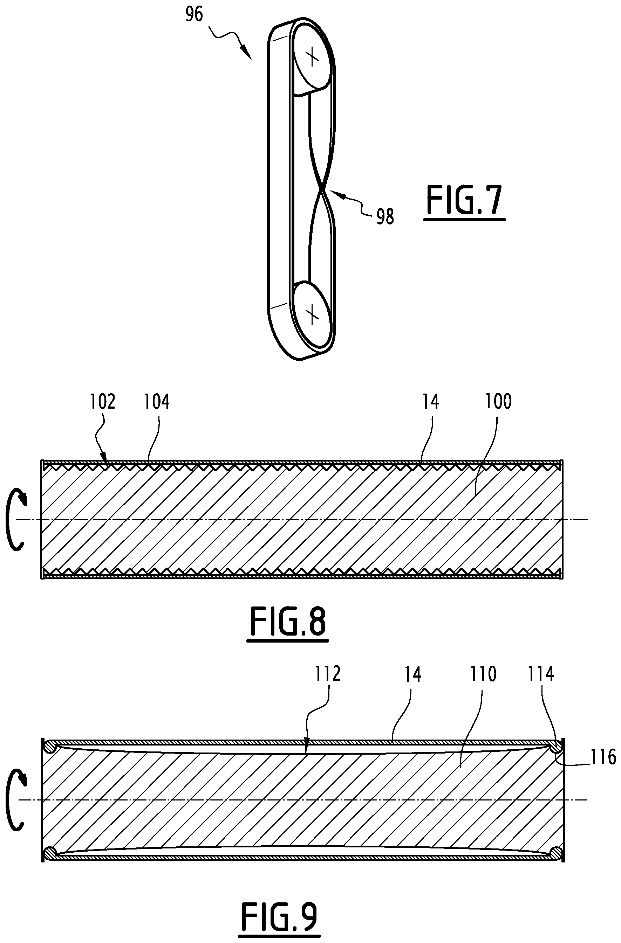

Dans chacun des modes de réalisation précédemment décrits, la croissance des micro-algues s'effectue principalement sur une unique face du support. En variante, et ceci est applicable à tous les modes de réalisation, le support, qui comprend un biofilm sur ses deux faces, est formé d'une bande de Möbius 96, de sorte que le support refermé en boucle comporte une face unique du fait du retournement de la bande autour de son axe de circulation dans un tronçon 98 comme illustré sur la figure 7. Ainsi, la croissance des micro-algues s'effectue sur toute la surface du support, le support se trouvant présenté sous une orientation différente lors de chaque passage sur le ou les tronçons d'exposition. Avantageusement, le tronçon de retournement de la bande s'effectue sur un tronçon de repos, permettant une exposition complète du support sur le ou les tronçons d'exposition.

Pour chacun des modes de réalisation précédents, en variante, l'installation comporte des moyens d'injection dans le milieu aqueux de nutriments et/ou de dioxyde de carbone, permettant un enrichissement du milieu en nutriments et/ou dioxyde de carbone pendant la circulation du support et la croissance du biofilm.

En variante, l'invention ne met pas en oeuvre seulement un mécanisme de photosynthèse pour la croissance du biofilm mais aussi un mécanisme de consommation du carbone organique, notamment des sucres et des acides organiques présent dans le milieu aqueux. Le procédé met alors en oeuvre la mixotrophie.

En variante, et quel que soit le mode de réalisation, le bassin est recouvert d'une serre transparente, permettant la circulation de l'air et évitant la contamination du milieu aqueux.

Sur la figure 8 est illustré un exemple de rouleau 100 de retournement du support circulant 14 en forme de bande. Afin de réduire la surface de contact entre le biofilm et le rouleau 100, la surface extérieure du rouleau comporte un profil extérieur en saillie et en creux formé par exemple d'un profil en hélice jointive 102 s'étendant suivant toute la surface généralement cylindrique du rouleau. En variante, la surface est pourvue de gorges accolées parallèles les unes aux autres, chaque gorge étant disposée transversalement à l'axe du rouleau.

On conçoit qu'avec un tel profil, le biofilm, lorsqu'il est en contact avec la surface extérieure du rouleau 100, n'entre en contact que suivant des lignes de contact ponctuelles notées 104, réduisant ainsi l'éventuelle détérioration du biofilm par le rouleau.

Dans le mode de réalisation alternatif illustré sur la figure 9, le rouleau noté 110 présente une surface extérieure concave 112 de sorte que le diamètre du rouleau augmente progressivement de sa partie médiane vers ses extrémités opposées. Le support 14 en forme de bande comporte latéralement, suivant toute sa longueur de part et d'autre, des bourrelets longitudinaux 114 reçus dans des gorges annulaires 116 ménagées aux deux extrémités du rouleau 110. Dans ces conditions, la partie médiane de la bande 114 est maintenue tendue entre les deux bourrelets 116 retenus dans les gorges 114.

Du fait de la forme en creux de la surface latérale du rouleau 110, la plage principale du support 14 reste maintenue à l'écart de la surface du rouleau, évitant ainsi tout risque de détérioration du biofilm formé sur la surface du support en regard du rouleau.

## Revendications

1. Procédé de production de micro-algues sur un support (14) monté mobile essentiellement dans un milieu aqueux contenu dans un bassin (12) comportant :
- une succession de phases d'exposition à la lumière solaire des micro-algues se développant sur le support (14) et de phases de séjour dans l'ombre, l'intensité lumineuse reçue dans l'ombre étant de moins de 50% de l'intensité lumineuse moyenne reçue lors des phases d'exposition à la lumière solaire, **caractérisé en ce que** la durée totale des phases de séjour dans l'ombre est supérieure de 50% à la durée totale des phases d'exposition à la lumière solaire.

2. Procédé de production de micro-algues selon la revendication 1, **caractérisé en ce que** la durée totale des phases de séjour dans l'ombre est comprise entre 2 et 10 fois la durée totale des phases d'exposition à la lumière.

3. Procédé de production de micro-algues selon la revendication 1 ou 2, **caractérisé en ce que** la durée de chaque phase d'exposition à la lumière est comprise entre 5 secondes et 10 minutes.

4. Procédé de production de micro-algues selon l'une quelconque des revendications précédentes, **caractérisé en ce que** le support (14) circule en boucle entre des rouleaux, et **en ce que** la vitesse du support (14) est comprise entre 1 cm/s et 1 m/s.

5. Procédé de production de micro-algues selon l'une quelconque des revendications précédentes, **caractérisé en ce que** la dose totale d'éclairement des micro-algues pendant chaque phase d'exposition est comprise entre 1 et 60 millimoles photons/m².

6. Procédé de production de micro-algues selon l'une quelconque des revendications précédentes, **caractérisé en ce que** les micro-algues font partie de l'une ou plusieurs des espèces comprises dans le groupe consistant en *Botryoccocus sp., Porhyridium sp., Cylindrotheca sp., Navicula sp. Haslea sp. et Chlorella sp,.*

7. Procédé de production de micro-algues selon l'une quelconque des revendications précédentes, **caractérisé en ce que** les micro-algues sont maintenues au moins 90% du temps dans le milieu aqueux.

8. Installation de production de micro-algues comportant :
- un bassin (12) ;
- un support (14) monté mobile dans un milieu aqueux contenu dans le bassin (12) ; et
- des moyens d'entrainement et de guidage du support (14) successivement sur des tronçons d'exposition (24 ; 60 ; 74) des micro-algues sur le support (14) à la lumière solaire et sur des tronçons de repos (26) dans l'ombre, l'agencement étant tel que l'intensité lumineuse reçue lors des déplacements sur les tronçons de repos dans l'ombre est de moins de 50% de l'intensité lumineuse moyenne reçue lors des déplacements sur les tronçons d'exposition à la lumière solaire, **caractérisée en ce que** les moyens d'entrainement et de guidage du support (14) sont adaptés pour que la durée totale des déplacements sur les tronçons de repos dans l'ombre est supérieure de 50% à la durée totale des déplacements sur les tronçons d'exposition (24 ; 60 ; 74) à la lumière solaire.

9. Installation de production de micro-algues selon la revendication 8, **caractérisée en ce que** le support (14) est replié en boucle et **en ce que** le support (14) est guidé suivant des tronçons (24, 26) superposés, le tronçon supérieur formant un tronçon d'exposition (24) disposé au-dessus des autres tronçons formant des tronçons de repos (26) situés dans l'ombre du tronçon d'exposition (24).

10. Installation de production de micro-algues selon la revendication 8, **caractérisée en ce que** le support (14) est replié en boucle et **en ce qu'**il comporte des moyens de guidage du support suivant des tronçons verticaux ascendants et descendants sensiblement perpendiculairement à la surface (16) du milieu aqueux contenu dans les bassins disposés dans l'ombre.

11. Installation de production de micro-algues selon l'une quelconques des revendications 8 à 10, **caractérisée en ce que** le support est une bande de Möbius (96).

## Patentansprüche

1. Verfahren zur Herstellung von Mikroalgen auf einem Träger (14), der im Wesentlichen in einem wässrigen Medium beweglich montiert ist, das in einem Becken (12) enthalten ist, beinhaltend:
- eine Abfolge von Sonnenlicht-Belichtungsphasen von Mikroalgen, die auf dem Träger (14) wachsen, und von Phasen des Aufenthalts im Schatten, wobei die im Schatten empfangene Lichtintensität weniger als 50 % der durchschnittlichen Lichtintensität beträgt, die während der Sonnenlicht-Belichtungsphasen empfangen wird, **dadurch gekennzeichnet, dass** die Gesamtdauer der Phasen des Aufenthalts im Schatten 50 % länger als die Gesamtdauer der Sonnenlicht-Belichtungsphasen ist.

2. Verfahren zur Herstellung von Mikroalgen nach Anspruch 1, **dadurch gekennzeichnet, dass** die Gesamtdauer der Phasen des Aufenthalts im Schatten zwischen dem 2- und 10-fachen der Gesamtdauer der Belichtungsphasen liegt.

3. Verfahren zur Herstellung von Mikroalgen nach Anspruch 1 oder 2, **dadurch gekennzeichnet, dass** die Dauer jeder Belichtungsphase im Bereich zwischen 5 Sekunden und 10 Minuten liegt.

4. Verfahren zur Herstellung von Mikroalgen nach einem der vorstehenden Ansprüche, **dadurch gekennzeichnet, dass** der Träger (14) in einer Schleife zwischen Rollen zirkuliert und **dadurch, dass** die Geschwindigkeit des Trägers (14) im Bereich zwischen 1 cm/s und 1 m/s liegt.

5. Verfahren zur Herstellung von Mikroalgen nach einem der vorstehenden Ansprüche, **dadurch gekennzeichnet, dass** die Gesamtbeleuchtungsdosis der Mikroalgen während jeder Belichtungsphase im Bereich zwischen 1 und 60 Millimol Photonen/m² liegt.

6. Verfahren zur Herstellung von Mikroalgen nach einem der vorstehenden Ansprüche, **dadurch gekennzeichnet, dass** die Mikroalgen zu einer oder mehreren Arten gehören, die in der Gruppe, bestehend aus *Botryoccocus sp., Porhyridium sp., Cylindrotheca sp., Navicula sp. Haslea sp. und Chlorella sp.,* umfasst sind.

7. Verfahren zur Herstellung von Mikroalgen nach einem der vorstehenden Ansprüche, **dadurch gekennzeichnet, dass** die Mikroalgen zu mindestens 90 % der Zeit in dem wässrigen Medium gehalten werden.

8. Anlage zur Herstellung von Mikroalgen, beinhaltend:
- ein Becken (12);
- einen Träger (14), der beweglich in einem wässrigen Medium montiert ist, das in dem Becken (12) enthalten ist; und
- Mittel zum sukzessiven Antreiben und Führen des Trägers (14) auf Sonnenlicht-Belichtungsabschnitte (24; 60; 74) der Mikroalgen auf dem Träger (12) und auf Ruheabschnitte (26) im Schatten, wobei die Anordnung derart ist, dass die Lichtintensität, die während der Bewegungen auf den Ruheabschnitten im Schatten empfangen wird, weniger als 50 % der durchschnittlichen Lichtintensität beträgt, die während der Bewegungen auf den Sonnenlicht-Belichtungsabschnitten empfangen wird, **dadurch gekennzeichnet, dass** die Mittel zum Antreiben und Führen des Trägers (14) so angepasst sind, dass die Gesamtdauer der Bewegungen auf den Ruheabschnitten im Schatten 50 % länger als die Gesamtdauer der Bewegungen auf den Sonnenlicht-Belichtungsabschnitten (24; 60; 74) ist.

9. Anlage zur Herstellung von Mikroalgen nach Anspruch 8, **dadurch gekennzeichnet, dass** der Träger (14) in einer Schleife verläuft und **dadurch, dass** der Träger (14) entlang übereinanderliegender Abschnitte (24, 26) geführt wird, wobei der obere Abschnitt einen Belichtungsabschnitt (24) bildet, der über den anderen Abschnitten angeordnet ist, die Ruheabschnitte (26) bilden, die im Schatten des Belichtungsabschnitts (24) angebracht sind.

10. Anlage zur Herstellung von Mikroalgen nach Anspruch 8, **dadurch gekennzeichnet, dass** der Träger (14) in einer Schleife verläuft und dadurch, dass sie Mittel zum Führen des Trägers entlang vertikal auf- und absteigender Abschnitte beinhaltet, die im Wesentlichen senkrecht zur Oberfläche (16) des wässrigen Mediums stehen, das in dem im Schatten angeordneten Becken enthalten ist.

11. Anlage zur Herstellung von Mikroalgen nach einem der vorstehenden Ansprüche 8 bis 10, **dadurch gekennzeichnet, dass** der Träger ein Möbiusband (96) ist.

## Claims

1. A method for producing microalgae on a support (14) movably mounted essentially in an aqueous medium contained in a tank (12) comprising:
- successive phases of exposing the microalgae growing on the support (14) to sunlight and phases of said microalgae residing in the shade, the light intensity received in the shade being less than 50% of the average light intensity received during the phases of exposure to sunlight, **characterized in that** the total duration of the phases of residing in the shade is 50% greater than the total duration of the phases of exposure to sunlight.

2. The method for producing microalgae as claimed in claim 1, **characterized in that** the total duration of the phases of residing in the shade is between 2 and 10 times the total duration of the phases of exposure to light.

3. The method for producing microalgae as claimed in claim 1 or 2, **characterized in that** the duration of each phase of exposure to light is between 5 seconds and 10 minutes.

4. The method for producing microalgae as claimed in any one of the preceding claims, **characterized in that** the support (14) circulates in a loop between rollers, and **in that** the speed of the support (14) is between 1 cm/s and 1 m/s.

5. The method for producing microalgae as claimed in any one of the preceding claims, **characterized in that** the total dose of illumination of the microalgae during each exposure phase is between 1 and 60 millimol photons/m².

6. The method for producing microalgae as claimed in any one of the preceding claims, **characterized in that** the microalgae are part of one or more of the species from the group consisting of *Botryoccocus sp., Porhyridium sp., Cylindrotheca sp., Navicula sp., Haslea sp.* and *Chlorella sp..*

7. The method for producing microalgae as claimed in any one of the preceding claims, **characterized in that** the microalgae are kept in the aqueous medium at least 90% of the time.

8. A unit for producing microalgae comprising:
- a tank (12);
- a support (14) movably mounted in an aqueous medium contained in the tank (12); and
- means for driving and guiding the support (14) successively over sections for exposing (24; 60; 74) the microalgae on the support (14) to sunlight and over sections for resting (26) in the shade, the arrangement being such that the light intensity received during travel on the sections for resting in the shade is less than 50% of the average light intensity received during travel on the sections for exposure to sunlight, **characterized in that** the means for driving and guiding the support (14) are set up such that the total duration of travel on the sections for resting in the shade is 50% greater than the total duration of travel on the sections for exposure (24; 60; 74) to sunlight.

9. The unit for producing microalgae as claimed in claim 8, **characterized in that** the support (14) is folded into a loop and **in that** the support (14) is guided along superposed sections (24, 26), the upper section forming an exposure section (24) arranged above the other sections forming rest sections (26) located in the shadow of the exposure section (24).

10. The unit for producing microalgae as claimed in claim 8, **characterized in that** the support (14) is folded into a loop and **in that** it comprises means for guiding the support along vertical rising and falling sections substantially perpendicular to the surface (16) of the aqueous medium which is contained in the tanks which are arranged in the shade.

11. The unit for producing microalgae as claimed in any one of claims 8 to 10, **characterized in that** the support is a Möbius strip (96).
